# FASCICULE DE BREVET EUROPEEN

(11) **EP 0 973 509 B1**
(45) Date de publication et mention de la délivrance du brevet: **07.04.2004**
(21) Numéro de dépôt: 98920577.8
(22) Date de dépôt: 07.04.1998
(51) Int. Cl.: A61K 9/26, A61K 9/16, A61K 9/52

(54) **FORME PHARMACEUTIQUE MULTIPARTICULAIRE, SES PARTICULES CONSTITUTIVES, PROCEDE ET INSTALLATION POUR LEUR FABRICATION**
MULTIPARTIKULÄRE PHARMAZEUTISCHE FORM, DIESE BILDENDE PARTIKELN, VERFAHREN UND VORRICHTUNG ZU DEREN HERSTELLUNG
MULTIPARTICULATE PHARMACEUTICAL FORM, PARTICLES CONSTITUTING IT, METHOD AND PLANT FOR MAKING SAME

(30) Priorité: 07.04.1997 FR 9704234
(43) Date de publication de la demande: 26.01.2000
(73) Titulaire: ETHYPHARM, 78550 Houdan (FR)
(72) Inventeur: LOUVEL, Pascal, Claude, Michel, F-93330 Neuilly sur Marne (FR); RAGOT, Françoise, F-28300 Leves (FR); CLEE, Marie-Christine, F-28500 Treon (FR); CHAUVEAU, Charles, F-06560 Valbonne (FR)
(74) Mandataire: Koch, Gustave
(86) Numéro de dépôt international: PCT/FR1998/000697
(87) Numéro de publication internationale: WO 1998/044911

(56) Documents cités:
- CH-A- 685 285
- DE-A- 4 122 217
- DE-A- 4 413 350

## Description

L'invention a pour objet une forme pharmaceutique multiparticulaire, notamment un comprimé multiparticulaire du genre de ceux qui sont à base d'une pluralité de particules individuelles obtenues par extrusion à chaud et contenant individuellement la matière active.

Elle vise également, en tant que produit industriel nouveau, les particules constitutives de la susdite forme pharmaceutique multiparticulaire.

Elle vise enfin un procédé et une installation pour la préparation desdites particules constitutives.

On connaît déjà des formes pharmaceutiques solides préparées à partir d'un mélange fondu sans solvant de substances actives et de matières thermoplastiques, notamment par le brevet US-A-3 432 592 selon lequel la mise en forme a lieu par moulage par injection et par le brevet EP-A-0 190 255 selon lequel la masse fondue est traitée soit par moulage par injection, soit par extrusion.

On connaît également, par les documents WO-A-9614058 et WO-A-9614059, des comprimés du genre en question qui sont préparés par compression d'un ensemble de particules individuelles constituées d'une matrice en matériau thermoplastique au sein duquel est dispersée une substance active, ces particules étant obtenues par découpage du fil sortant de la filière d'une extrudeuse à l'intérieur de laquelle le mélange du matériau thermoplastique et de la substance active a auparavant été soumis aux conditions d'une extrusion à chaud; il est à noter qu'avant l'étape de compression les particules obtenues à la sortie de la filière peuvent être soumises à une sphéronisation afin de leur conférer des contours arrondis.

L'inconvénient principal de ces comprimés réside notamment dans leur faible vitesse de délitement qui rend très difficile la dispersion des particules individuelles dans le tractus digestif.

On connaît de plus, par le document EP-A-0 548 356, des comprimés du genre en question préparés par compression d'un mélange comprenant, d'une part, des particules individuelles obtenues par extrusion-sphéronisation d'une substance active et pourvues d'un enrobage de masquage du goût et, d'autre part, un excipient à base d'au moins un agent désintégrant et/ou d'au moins un agent gonflant.

Ces comprimés se distinguent par une très grande vitesse de délitement conduisant, au niveau de la cavité buccale, à la dispersion des particules individuelles, ce qui est rendu acceptable grâce à la présence de leur enrobage.

On connaît enfin
- par le document DE-A-44 13 350, des comprimés à base de particules obtenues par extrusion à chaud à partir du mélange d'une substance active et d'un polymère avec découpage du fil extrudé et mise en forme des particules, ces particules étant éventuellement pourvues d'un enrobage constitué par un polyacrylate ou un ester de cellulose,
- par le document DE-A-41 22 217, des comprimés à base de particules obtenues par extrusion humide à froid à partir d'un mélange de polymères et de substance active, suivie d'une sphéronisation et d'un enrobage.

L'invention a pour but, surtout, de fournir une forme pharmaceutique multiparticulaire, notamment un comprimé multiparticulaire du genre en question, qui soit douée d'une vitesse de délitement telle que son délitement et, par conséquent, la dispersion des particules constitutives individuelles, se produisent en moins de 15 minutes dans un désagrégomètre standard, en d'autres termes dans la partie haute du tube digestif.

Et il est du mérite de la Société Demanderesse d'avoir trouvé, à l'issue de recherches approfondies, que ce but était atteint dès lors que les particules individuelles constitutives de ladite forme pharmaceutique multiparticulaire comportent un revêtement à base d'au moins un agent liant, d'au moins un agent désintégrant et éventuellement d'au moins un principe actif.

Il s'ensuit que la forme pharmaceutique multiparticulaire est caractérisée par le fait qu'elle est constituée par une pluralité de particules, de préférence de sphéroïdes comportant un enrobage à base d'au moins un agent désintégrant et d'au moins un agent liant et éventuellement d'au moins une substance active et solidarisés les uns avec les autres par compression directe lesdits particules ou sphéroïdes dans lesquels une substance active est répartie au sein d'un matériau thermoplastique ayant été obtenus par extrusion à chaud sans solvant du mélange de la substance active avec le matériau thermoplastique et découpage du fil ou profilé sortant de la filière d'extrusion.

Les particules, de préférence les sphéroïdes, constitutives de la forme pharmaceutique multiparticulaire, sont caractérisées par le fait qu'elles sont le résultat du découpage du fil ou profilé obtenu par extrusion à chaud sans solvant du mélange d'une substance active et d'un matériau thermoplastique et de l'enrobage des particules résultant du découpage par un revêtement comportant au moins un agent liant et.au moins un agent désintégrant.

C'est en raison de l'extraction progressive de la substance active hors de la matrice constituée par le matériau thermoplastique --extraction qui fait passer la substance active dans le milieu environnant-- que les formes pharmaceutiques multiparticulaires, notamment les comprimés multiparticulaires conformes à l'invention constituent une forme pharmaceutique à libération modifiée et contrôlée.

La courbe traduisant la libération in vitro exprimée en % de la substance active en fonction du temps est une caractéristique essentielle prédéterminée de la forme pharmaceutique et doit se reproduire d'un comprimé à l'autre.

Elle est le résultat du choix judicieux des caractéristiques constitutives de la matrice.

Dans le cas de la préparation des particules individuelles entrant dans la constitution de la forme pharmaceutique multiparticulaire, notamment du comprimé multiparticulaire, par extrusion du mélange d'une substance active et d'un matériau thermoplastique constitutif de la matrice et par découpage du fil extrudé à la sortie de la filière, il importe que le traitement d'extrusion soit effectué en moins de 6 heures à 25°C après la constitution du mélange à extruder sous peine d'obtenir des particules et partant des comprimés pour lesquels la courbe traduisant la libération de la substance active en fonction du temps se trouve modifiée dans le sens d'une diminution du pourcentage de substance active libérée par unité de temps.

Cette contrainte qui n'est pas décrite dans l'art antérieur constitue un inconvénient majeur en ce sens que, si pour une raison quelconque un mélange prêt pour l'extrusion devait être stocké pendant un certain temps supérieur à la limite indiquée ci-dessus avant de pouvoir être extrudé, ce mélange devra être considéré comme perdu en raison de l'inévitable altération de la courbe de libération de la substance active à partir des particules issues de l'extrusion.

Or, la Société Demanderesse a le mérite d'avoir trouvé qu'il était possible de remédier à cet inconvénient en soumettant à une étape de maturation le mélange de la substance active et du matériau thermoplastique.

En effet, dès lors que ledit mélange a été soumis à cette étape de maturation --dont la durée est de 30 minutes à 150 heures et la température de 20 à 70°C--, son extrusion fournit des particules donnant lieu à une courbe de libération du principe actif qui est stabilisée sous réserve que le laps de temps séparant la constitution du mélange et son extrusion n'excède pas environ 7 jours.

Il se trouve que cette courbe --stabilisée-- de libération du principe actif traduit une cinétique plus lente que celle obtenue avec des particules de même composition mais extrudées immédiatement après constitution du mélange, sans étape de maturation; cela signifie qu'à un instant donné le pourcentage de substance active libérée par les particules préparées par extrusion après maturation est inférieur à celui libéré par les particules extrudées sans maturation.

Pour faire en sorte que les deux courbes se superposent, il convient donc de concentrer davantage en substance active le mélange soumis à une étape de maturation avant extrusion.

La phase de maturation entraîne par conséquent un avantage supplémentaire qui réside dans le fait que les particules obtenues par le procédé conforme à l'invention comportant cette phase de maturation apportent à l'organisme et à vitesse équivalente une quantité accrue de substance active pour un volume inchangé, voire diminué.

Il s'ensuit que le procédé de fabrication conforme à l'invention de particules, notamment de sphéroïdes de diamètre constant compris entre 0,5 et 2 mm, comportant une substance active et présentant une courbe de libération de la substance active contrôlée et prédéterminée et qui comporte successivement:
- une étape de sélection d'une substance active et d'un matériau thermoplastique, comportant au moins un excipient de nature polymère et au moins un agent plastifiant,
- une étape de mélange de la substance active et du matériau thermoplastique,
- une étape d'extrusion sans solvant du mélange à température contrôlée fournissant au moins un fil ou profilé extrudé,
- une étape de découpage du profilé extrudé en particules élémentaires,
est caractérisé par le fait qu'il comporte une étape de mûrissement du mélange de la substance active et du matériau thermoplastique avant extrusion à une température et pendant une durée choisies en fonction des caractéristiques de la courbe de libération du principe actif visé comprises respectivement entre 20 et 70°C et entre 30 minutes et 150 heures, ce grâce à quoi il devient possible, d'une part, de stocker le mélange de substance active et de matériau thermoplastique jusqu'à 7 jours avant l'extrusion et, d'autre part, d'administrer à une vitesse de libération donnée une quantité accrue de substance active sans augmentation de volume du comprimé.

Ainsi qu'il résulte de ce qui précède, on préfère que les particules constitutives de la forme pharmaceutique multiparticulaire, notamment du comprimé multiparticulaire se présentent sous forme de sphéroïdes, c'est-à-dire présentent des contours arrondis.

Or, la sphéronisation constitue une étape supplémentaire génératrice d'un surcoût; de plus, elle n'est possible que si le profilé conserve des propriétés physiques adéquates pour être mis en forme sphéroïdale à la température de travail.

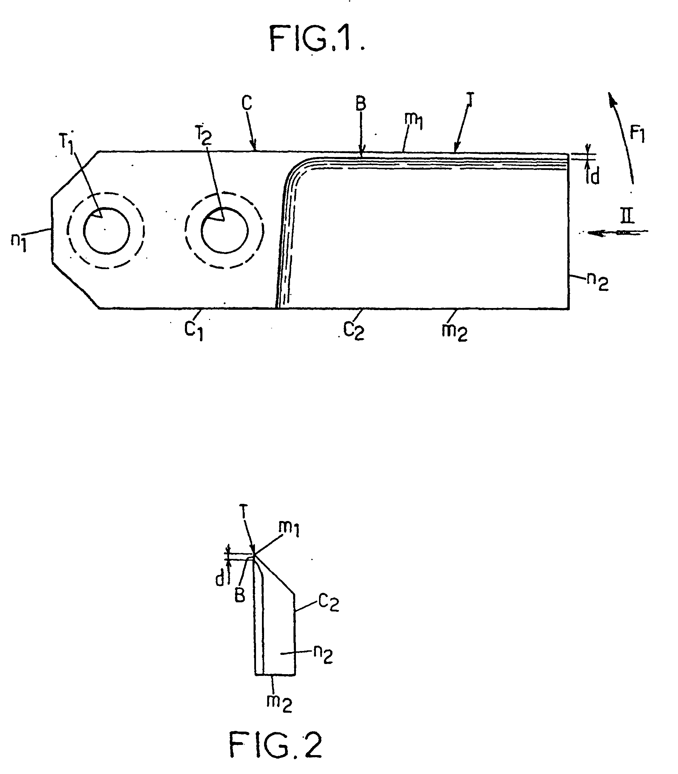

La Société Demanderesse a eu le mérite de trouver qu'il était possible d'obtenir directement sous forme sphéroïdale, à la sortie de la filière, les particules dès lors que les couteaux comportés par l'outil de coupe disposé à la sortie de la filière présentent la forme qui résulte des figures 1 et 2 et qui sera décrite plus loin.

Il s'ensuit que l'installation conforme à l'invention pour la fabrication des sphéroïdes conformes à l'invention, est constituée par une extrudeuse comportant, à la sortie de la filière d'extrusion, un outil de coupe du fil ou profilé extrudé équipé de couteaux présentant la forme qui résulte des figures 1 et 2.

L'invention pourra être encore mieux comprise à l'aide du complément de description qui suit et des exemples non limitatifs qui sont relatifs à des modes de réalisation avantageux.

Se proposant, par conséquent, de constituer les particules, notamment les sphéroïdes, conformes à l'invention ainsi que la forme pharmaceutique multiparticulaire, notamment le comprimé multiparticulaire les contenant, on s'y prend comme suit ou de façon équivalente.

On sélectionne tout d'abord une substance active et les constituants d'un matériau thermoplastique, à savoir au moins un polymère et au moins un agent plastifiant.

La substance active qui doit être stable, du point de vue physique et du point de vue chimique, à la température de ramollissement du matériau thermoplastique, est avantageusement choisie dans le groupe comprenant les sédatifs gastro-intestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les anti-diarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

Sa granulométrie doit être compatible avec une dispersion homogène au sein du matériau thermoplastique.

Elle est avantageusement de 0,1 à 500 µm et, de préférence, de 1 à 100 µm.

Le polymère est un polymère thermoplastique dont la température de ramollissement se situe de préférence de 10 à 50°C au-dessous de la température de fusion de la substance active; il est avantageusement choisi dans le groupe comprenant les éthylcelluloses, les hydroxypropylcelluloses, les hydroxyéthylcelluloses, les hydroxypropylméthylcelluloses, les acétosuccinates d'hydroxypropylméthylcellulose, les acétobutyrates de cellulose, les acétopropionates de cellulose, les acétophtalates de cellulose, les phtalates d'hydroxypropylméthylcellulose, les celluloses microcristallines, les carboxyméthylcelluloses sodiques, les polyméthacrylates, les oxydes de polyéthylène, les polyvinylpyrrolidones, les amidons réticulés, l'acide lactique, les polyvinylacétates, les polyvinyles alcools et les résines d'acétate de vinyle.

Il se présente avantageusement sous la forme d'une poudre.

Le plastifiant est choisi de préférence parmi ceux qui sont liquides et plus particulièrement dans le groupe comprenant le phtalate d'éthyle, le dibutylsébacate, la triacétine, le triéthyl-citrate, le dioctyl-phtalate, le diphényl-phtalate, le dibutyl-phtalate, les polyéthylène-glycols et les monoglycérides acétylés.

On mélange les poudres de substance active et de polymère thermoplastique et on incorpore au mélange l'agent plastifiant.

Pour effectuer ce mélange, on peut avoir recours à un mélangeur planétaire ou préférentiellement à un mélangeur à cisaillement rapide à double enveloppe.

On peut utiliser les mélangeurs commercialisés par la Société Collette.

La température des constituants du mélange est de 20 à 70°C au moment de l'opération de mélange.

Les proportions respectives de substance active, de polymère et d'agent plastifiant sont de préférence comme suit:
- substance active 5 à 80% en poids
- polymère 5 à 80% en poids
- agent plastifiant 1 à 25% en poids.

Avantageusement on fait comporter au mélange une proportion de colorant de 0,1 à 1% en poids.

C'est le mélange ainsi obtenu qui est soumis à une opération d'extrusion.

Mais auparavant, conformément à l'invention, on lui fait subir une étape de maturation.

L'étape de maturation consiste à maintenir le mélange au repos pendant une durée de 30 minutes à 150 heures à une température de 20 à 70°C, de préférence de 35 à 70°C, avantageusement à l'intérieur d'une étuve ventilée à plateaux.

La durée de l'étape de maturation peut être réduite à moins de 10 heures à condition de maintenir le mélange pendant l'étape de maturation non pas au repos mais sous agitation lente dans le mélangeur.

Grâce à cette étape de maturation, le mélange constitué par le matériau thermoplastique et la substance active peut être stocké jusqu'à 7 jours avant l'étape d'extrusion.

Celle-ci est effectuée dans une extrudeuse, par exemple monovis, dans la zone de malaxage de laquelle elle est introduite sous forme pulvérulente.

A l'intérieur de la zone de malaxage, le mélange est soumis aux conditions de température et de pression suivantes:
- température : entre 20 et 200°C
- pression : entre 10 et 160 bars.

La durée du séjour à l'intérieur de la zone de malaxage est de 2 à 6 minutes.

Les paramètres de température, de pression et de durée sont choisis en fonction des caractéristiques du polymère du mélange et de la taille des sphéroïdes que l'on souhaite obtenir.

Sous les conditions régnant dans la zone de malaxage, le mélange pulvérulent de départ est transformé en une pâte fluide au sein de laquelle les particules de substance active restent à l'état solide.

La pâte fluide est extrudée au travers d'une filière dont le diamètre, qui est fonction de la taille des sphéroïdes que l'on souhaite obtenir, est de 0,5 à 2,0 mm.

A la sortie de la filière, le mélange extrudé se présente sous la forme d'un fil ou d'un profilé dont le diamètre excède le diamètre de la filière de 20 à 150% par suite d'une expansion; ce fil ou profilé est solidifié et rigidifié par refroidissement, par exemple sous l'influence d'un jet d'air.

Il est possible d'utiliser une filière multiple qui fournit plusieurs fils ou profilés.

La vitesse de sortie du fil est de 10 à 80 g/minute; cette vitesse est fonction de la vitesse de rotation de la vis.

Le fil ou profilé obtenu à la sortie de la filière est découpé à l'aide d'un outil de coupe rotatif comportant un ou plusieurs couteaux; l'outil de coupe est disposé de telle sorte que la distance entre la face de la filière devant l'orifice de laquelle se déplacent les couteaux de par la rotation de l'outil et la surface desdits couteaux, est de 0,04 à 0,15 mm.

Conformément à l'invention, les couteaux présentent la forme qui résulte des figures 1 et 2.

La figure 1 est une vue en plan d'un couteau C agencé conformément à l'invention et la figure 2 est une vue en bout suivant II-II de la figure 1.

Comme montré à la figure 1, le couteau C, qui est de forme générale rectangulaire dont les deux grands côtés sont désignés par m₁ et m₂, les deux petits côtés étant désignés par n₁ et n₂, comporte:
- une partie C₁ pleine par laquelle le couteau est fixé sur l'outil de coupe non montré, par exemple par des vis non montrées pour le logement desquelles il a été,prévu deux trous taraudés T₁ et T₂, et
- une partie C₂ évidée à partir du grand côté m₂ en direction du grand côté m₁ qui comporte le tranchant T du couteau jusqu'à une distance d de ce côté m₁ qui est inférieure à 1 mm, de telle sorte que la surface du couteau qui est représentée par la face montrée de la partie C₁ se prolonge, au niveau de la partie C₂, le long du côté m₁ par une bande B de largeur d.

La forme évidée de la partie C₂, le tranchant comporté par le côté m₁ et la bande B de largeur d apparaissent clairement sur la figure 2.

Le sens du déplacement du couteau lors de la rotation de l'outil de coupe est montré par la flèche F₁ sur la figure 1.

Il en résulte que, lors de la rotation de l'outil de coupe, le couteau C heurte le fil non montré sortant de la filière non montrée par le tranchant T et provoque ainsi le découpage du fil en particules successives.

C'est grâce au fait que, conformément à l'invention, la largeur de la bande B est inférieure à 1 mm que le découpage du fil ou profilé sortant de la filière fournit directement et sans étape de sphéronisation supplémentaire, des particules de forme sphéroïde, la valeur précise de d étant déterminée en fonction du diamètre du trou de la filière et de la vitesse avec laquelle le fil extrudé sort de cette dernière.

La relation entre la valeur de d, le diamètre du trou de la filière et la vitesse de sortie du profilé est déterminée empiriquement au cas par cas.

La plus grande dimension des particules sphéroïdales obtenues est généralement de 0,5 à 2 mm.

Cette dimension est une fonction de la vitesse de rotation de l'arbre de la vis d'extrusion, du gradient de température régnant dans la zone d'extrusion, de la température et des dimensions de la filière. La vitesse de rotation de l'arbre de la vis est de préférence de 1 à 90 tours par minute. Le gradient de température dans la zone d'extrusion et la température de la filière sont de préférence compris entre 20 et 200°C. Le diamètre de l'orifice de la filière est de préférence compris entre 0,5 et 2 mm.

La vitesse de rotation de l'outil de coupe est fixée en fonction de la vitesse à laquelle le fil extrudé sort de l'orifice de la filière; de préférence, elle est de 40 à 1500 tours/minute.

Les particules en forme de sphéroïdes, obtenues à la sortie de la filière, sont enrobées d'un revêtement comportant au moins un agent désintégrant; ce revêtement peut également comporter un agent liant; il peut enfin contenir une substance active.

pour ce faire, on peut avoir recours à une turbine ou à une installation à lit d'air fluidisé.

Tout d'abord, on pulvérise l'agent liant sous forme d'une solution de 10 à 30% en poids dans un solvant approprié et pharmaceutiquement acceptable, de préférence l'alcool ou l'eau. Une quantité d'agent désintégrant correspondant à 1 à 50% en poids par rapport au poids de sphéroïdes mis en oeuvre est ensuite fixée à l'état pulvérulent sur le sphéroïde recouvert de solution liante.

La forme sphéroïdale de la particule est essentielle pour obtenir un enrobage efficace.

La granulométrie de l'agent désintégrant mis en oeuvre est de préférence de 1 à 100 µm.

L'agent désintégrant est choisi dans le groupe comprenant les polyvinylpyrrolidones réticulées, les amidons modifiés et les carboxyméthylcelluloses sodiques réticulées et l'agent liant de préférence dans le groupe comprenant la polyvinylpyrrolidone, les polyéthylène-glycols, les polyméthacrylates et les hydroxypropylméthylcelluloses.

La masse totale d'enrobage correspond de préférence à une quantité de 2 à 20% en poids par rapport à la masse des particules sphéroïdales.

La forme pharmaceutique multiparticulaire, notamment le comprimé multiparticulaire, conforme à l'invention est préparée à partir des sphéroïdes ainsi obtenus.

Pour ce faire, on a recours aux techniques de compression bien connues de l'homme du métier.

On règle les paramètres de l'étape de compression de façon telle que la dureté des comprimés résultants soit de 30 à 70 N.

### EXEMPLE 1

Cet exemple concerne des sphéroïdes comportant du diclofénac de sodium en tant que substance active.

Il comporte une étude de l'influence de l'étape de maturation sur la vitesse de libération de la substance active à partir de ces sphéroïdes.

En tant que polymère constitutif du matériau thermoplastique, on choisit un polymère d'éthylcellulose dont le point de ramollissement est de 130-133°C (température de transition vitreuse).

Ce polymère n'est pas ou peu soluble en milieu aqueux; la viscosité d'une solution alcoolique à 5% (m/m) du polymère est de 5 à 8 mPa à 25°C. Il s'agit de celui commercialisé sous la désignation EC N7NF par la Société Aqualon/Hercules.

En tant que plastifiant, on utilise le diéthyl-phtalate.

Dans ce qui suit, la substance active, le polymère et le plastifiant sont désignés par les abréviations suivantes:
- DFC : diclofénac sodique
- EC N7NF : polymère d'éthylcellulose
- DEP : diéthyl-phtalate.

La composition centésimale du mélange destiné à la préparation des sphéroïdes est comme suit:
- DFC : 25% en poids
- EC N7NF : 65% en poids
- DEP : 10% en poids.

Pour étudier l'influence de l'étape de maturation sur la courbe de libération de la substance active à partir des sphéroïdes obtenus par extrusion du susdit mélange, on a préparé neuf échantillons de ce mélange désignés respectivement par A1, A2, B1, B2, B3, B4, C1, C2 et C3.

Les échantillons A1 et A2 ont été maintenus, avant extrusion, à la température ambiante respectivement pendant 4 heures et 2 jours.

Les échantillons B1 à B4 ont été soumis à une étape de maturation à 70°C en étuve pendant respectivement 4 heures, 1 jour, deux jours et trois jours.

Les échantillons C1 à C3 ont été soumis à une étape de maturation à 40°C en étuve pendant respectivement 1 jour, 2 jours et 3 jours.

Les neuf échantillons ont ensuite été extrudés et découpés à l'aide d'une extrudeuse monovis, les paramètres d'extrusion étant:
- température dans la zone d'extrusion: 130 à 170°C
- vitesse de la vis: de 20 à 75 tours/minute
- pression régnant dans la zone d'extrusion: 25 à 130 bars
- diamètre de l'orifice de la filière: 1 mm.

Le fil ou profilé extrudé est découpé à l'aide d'un outil de coupe comportant des couteaux conformes à l'invention, la vitesse de rotation de l'outil de coupe étant réglée de façon à obtenir une sphéronisation à chaud la plus parfaite possible; cette vitesse est notamment de 756 tours/minute.

La granulométrie des sphéroïdes obtenus est de 1,2 à 1,6 mm.

En utilisant un dissolumètre correspondant à celui désigné sous l'appellation "Appareil I" dans la Pharmacopée Américaine (USP XXIII) et "Appareil à panier" dans la Pharmacopée Européenne (3e édition), les conditions d'utilisation de l'appareil étant de 1 litre de tampon à pH 7,0 et à 37°C par réacteur et une vitesse de rotation de 100 tours/minute, on a déterminé pour les sphéroïdes résultant de chacun des échantillons A1 à C3 traités comme indiqué ci-dessus, le pourcentage de substance active libérée in vitro après 0, 5 heure, 1 heure, 1,5 heure, 2 heures, 3 heures, 4 heures, 5 heures, 6 heures, 7 heures et 8 heures.

On a réuni dans le tableau I les valeurs enregistrées.

**TABLEAU I**

| H | Pourcentage de substance active libérée dans le cas des échantillons | | | | | | | | |
|---|---|---|---|---|---|---|---|---|---|
| | A1 | A2 | B1 | B2 | B3 | B4 | C1 | C2 | C3 |
| 0,5 heure | 31,5 | 29,6 | 12,4 | 8,8 | 6,0 | 4,7 | 17,7 | 10,6 | 9,1 |
| 1 heure | 44,1 | 41,1 | 22,9 | 20,2 | 12,7 | 9,7 | 30,6 | 19,8 | 17,8 |
| 1,5 heure | 53,2 | 50,8 | 31,7 | 32,3 | 21,3 | 16,9 | 41,9 | 28,1 | 26,1 |
| 2 heures | 62,8 | 57,4 | 39,4 | 41,6 | 29,4 | 23,9 | 49,7 | 35,6 | 32,6 |
| 3 heures | 72,8 | 63,5 | 54,5 | 59,0 | 42,6 | 36,3 | 62,9 | 49,9 | 44,9 |
| 4 heures | 77,1 | 74,5 | 63,3 | 68,4 | 54,2 | 47,5 | 70,1 | 57,7 | 55,0 |
| 5 heures | 82,7 | 79,4 | 69,8 | 74,1 | 61,5 | 55,7 | 73,1 | 64,9 | 61,1 |
| 6 heures | 85,6 | 82,3 | 71,9 | 75,5 | 66,9 | 62,0 | 76,1 | 69,0 | 66,5 |
| 7 heures | 87,3 | 85,5 | 74,7 | 77,2 | 71,7 | 66,2 | 80,3 | 72,5 | 70,2 |
| 8 heures | 86,5 | 85,1 | 76,2 | 78,7 | 72,9 | 69,5 | 80,4 | 75,2 | 72,5 |

Dans la colonne H, on identifie le moment de la mesure par le temps écoulé depuis le début de l'expérience.

Les conclusions qu'il est possible de tirer des valeurs réunies dans ce tableau sont que le traitement de maturation entraîne une modulation dans le sens d'un ralentissement de la cinétique de dissolution de la substance active.

### EXEMPLE 2

Cet exemple illustre la stabilisation, grâce à l'étape de maturation, de la courbe de libération ou de dissolution du principe actif à partir de sphéroïdes obtenus en utilisant le même mélange que celui décrit à l'exemple 1, c'est-à-dire constitué comme suit:
- DFC : 25% en poids
- EC N7NF : 65% en poids
- DEP : 10% en poids
lorsque le mélange maturé est stocké avant d'être extrudé.

On prépare trois échantillons, respectivement D1, D2 et D3, à partir de ce mélange.

L'échantillon D1 est maintenu pendant 4 heures à la température ambiante avant d'être extrudé, sans étape de maturation.

L'échantillon D2 est soumis à une étape de maturation de 2 jours à 40°C puis extrudé.

L'échantillon D3 est soumis à la même étape de maturation que l'échantillon D2 puis stocké à la température ambiante pendant 4 jours, puis extrudé.

Les caractéristiques du traitement d'extrusion sont celles exposées à l'exemple 1.

Les sphéroïdes obtenus ont une granulométrie de 1,2 à 1,6 mm.

En procédant comme à l'exemple 1, on a déterminé les pourcentages de substance active libérée in vitro après 0,5 heure , 1 heure, 1,5 heure, 2 heures, 3 heures, 4 heures, 5 heures, 6 heures, 7 heures et 8 heures.

On a réuni dans le tableau II les valeurs enregistrées.

**TABLEAU II**

| H | Pourcentage de substance active libérée dans le cas des échantillons | | |
|---|---|---|---|
| | D1 | D2 | D3 |
| 0,5 heure | 25,8 | 11,4 | 9,5 |
| 1 heure | 40,0 | 23,5 | 19,0 |
| 1,5 heure | 48,6 | 34,5 | 27,7 |
| 2 heures | 55,8 | 41,8 | 35,9 |
| 3 heures | 64,6 | 56,1 | 49,4 |
| 4 heures | 73,5 | 65,7 | 60,2 |
| 5 heures | 75,5 | 69,8 | 67,1 |
| 6 heures | 76,8 | 73,7 | 71,0 |
| 7 heures | 81,3 | 77,7 | 73,2 |
| 8 heures | 79,9 | 78,2 | 75,1 |

Les résultats réunis dans le tableau II montrent que les courbes de libération de la substance active sont stabilisées lorsque le mélange à partir duquel sont obtenus les sphéroïdes a été soumis à une étape de maturation avant l'extrusion, rendant ainsi possible le stockage avant extrusion d'un tel mélange.

### EXEMPLE 3

Cet exemple a pour objet un comprimé multiparticulaire à libération modifiée contenant du diclofénac de sodium en tant que substance active.

En vue de l'étape d'extrusion, on prépare le mélange pulvérulent suivant:
- DFC 40 % en poids
- EC N7NF 52 % en poids
- DEP 8 % en poids
- Colorant E110 0,2% en poids.

Ce mélange pulvérulent est extrudé sur une extrudeuse monovis.

Les paramètres d'extrusion sont:
- température de la zone de malaxage et de la filière: 130 à 170°C
- vitesse de la vis: 50 tours/minute
- pression régnant dans la zone de malaxage: 25 à 70 bars
- vitesse de rotation de l'outil de coupe: 746 tours/minute
- diamètre de l'orifice de la filière: 1 mm.

Les sphéroïdes obtenus sont utilisés pour la préparation de comprimés multiparticulaires dosés à 100 mg de DFC.

On procède d'abord à l'enrobage des sphéroïdes.

Pour ce faire, les sphéroïdes sont disposés dans une turbine.

On leur applique tout d'abord un liant constitùé par une solution alcoolisée de la polyvinylpyrrolidone commercialisée sous la désignation PVP K30 par la Société BASF.

Sur les sphéroïdes ainsi traités, on applique un agent désintégrant, à savoir celui commercialisé par la Société BASF sous la marque KOLLIDON CLM. On prépare ainsi trois échantillons de sphéroïdes sur lesquels on applique respectivement des doses de 10, 15 et 20% en poids d'agent désintégrant par rapport à la masse du sphéroïde.

A partir des trois échantillons de sphéroïdes ainsi constitués et d'un échantillon de sphéroïdes non enrobés, on prépare des comprimés multiparticulaires par compression sur une machine à comprimer rotative vendue sous la marque SVIAC PR 6 par la Société SVIAC en utilisant des poinçons chanfreinés de 10 mm de diamètre.

Pour les comprimés ainsi obtenus, on détermine:
- la masse moyenne,
- l'épaisseur,
- la dureté,
- la friabilité et
- le temps de désagrégation (en utilisant les indicàtions de la Pharmacopée Européenne 1997, 2.9.1.).

Les valeurs ainsi obtenues sont réunies dans le tableau III dans lequel on fait également figurer la masse théorique et la quantité de matière active par gramme de sphéroïde ou titre.

**TABLEAU III**

| | Sphéroïdes non enrobés | Sphéroïdes pelliculés 10% | Sphéroïdes pelliculés 15% | Sphéroïdes pelliculés 20% |
|---|---|---|---|---|
| Titre | 411,9 mg/g | 382,6 mg/g | 363,3 mg/g | 343,0 mg/g |
| Masse théorique | 242,78 mg | 261,37mg | 275,25 mg | 291,55 mg |
| Masse moyenne | 242,9 mg | 255,2 mg | 276,33 mg | 294,83 mg |
| C.V. masse | 2,43% | 2,31% | 1,90% | 2,14% |
| Dureté | 45,8 N | 41,9 N | 39,4 N | 37,8 N |
| Epaisseur | 3,15 mm | 3,21 mm | 3,53 mm | 3,86 mm |
| Friabilité | 0,02% | 0,55% | 0,18% | 0,36% |
| Temps de désagrégation | > 15 min | 6 min 40 s | 7 min 40 s | 12 min 30 s |

A l'examen des résultats réunis dans le tableau III, il apparaît que la vitesse de désagrégation du comprimé augmente avec la proportion d'agent désintégrant d'enrobage.

Pour obtenir la vitesse de désagrégation recherchée, on peut utiliser une proportion d'agent désintégrant inférieure à 20%.

### EXEMPLE 4

Cet exemple a pour objet un comprimé multiparticulaire à libération modifiée comportant, en tant que substance active, de la pseudoéphédrine.

On prépare le mélange suivant:
- Pseudoéphédrine 40 % en poids
- EC N7NF 52 % en poids
- DEP 8 % en poids.

A partir de ce mélange pulvérulent, on prépare des sphéroïdes en utilisant une extrudeuse monovis, les paramètres d'extrusion étant:
- température dans la zone d'extrusion et dans la filière: 130 à 170°C
- vitesse de la vis: 72 tours/minute
- pression régnant dans la zone d'extrusion: 40 à 90 bars
- vitesse de rotation de l'outil de coupe: 700 tours/minute
- diamètre de l'orifice de la filière: 1 mm.

Les sphéroïdes obtenus sont utilisés pour la préparation de comprimés multiparticulaires dosés à 240 mg de pseudoéphédrine.

On procède d'abord à l'enrobage des sphéroïdes.

Pour ce faire, les sphéroïdes sont disposés dans une turbine.

On leur applique tout d'abord un liant constitué par une solution alcoolisée de la polyvinylpyrrolidone commercialisée sous la désignation PVP K30 par la Société BASF.

Sur les sphéroïdes ainsi traités, on applique un agent désintégrant, à savoir celui commercialisé par la Société BASF sous la marque KOLLIDON CLM. On prépare ainsi quatre échantillons de sphéroïdes sur lesquels on applique respectivement des doses de 5, 10, 15 et 20% en poids d'agent désintégrant par rapport à la masse du sphéroïde.

A partir des quatre échantillons de sphéroïdes ainsi constitués et d'un échantillon de sphéroïdes non enrobés, on prépare des comprimés multiparticulaires par compression sur une machine à comprimer rotative vendue sous la marque SVIAC PR 6 par la Société SVIAC en utilisant des poinçons chanfreinés de 14 mm de diamètre.

Pour les comprimés ainsi obtenus, on détermine:
- la masse moyenne,
- l'épaisseur,
- la dureté,
- la friabilité et
- le temps de désagrégation (en utilisant les indications de la Pharmacopée Européenne 1997, 2.9.1.).

Les valeurs ainsi obtenues sont réunies dans le tableau IV dans lequel on fait également figurer la masse théorique et la quantité de matière active par gramme de sphéroïde ou titre.

**TABLEAU IV**

| | Sphéroïdes non enrobés | Sphéroïdes pelliculés 5% | Sphéroïdes pelliculés 10% | Sphéroïdes pelliculés 15% | Sphéroïdes pelliculés 20% |
|---|---|---|---|---|---|
| Titre | 384,6 mg/g | 354;8 mg/g | 332,4 mg/g | 322,5 mg/g | 308,2 mg/g |
| Masse théorique | 624,02 mg | 676,44 mg | 722,02 mg | 744,19 mg | 778,72 mg |
| Masse moyenne | 622,5 mg | 683,4 mg | 729,0 mg | 744,3 mg | 771,9 mg |
| C.V. masse | 1,37% | 1,21% | 1,17% | 1,36% | 1,18% |
| Dureté | 41,2 N | 41,1 N | 39,5 N | 28,1 N | 37,5 N |
| Epaisseur | 4,82 mm | 5,08 mm | 5,28 mm | 5,55 mm | 5,66 mm |
| Friabilité | 0,02% | 0,01% | 0,21% | 2 cp cassés | 4 cp cassés |
| Temps de désagrégation | > 15 min | 4 min 07 s | 13 min 27 s | 8 min 58 s | > 15 min |

A l'examen des résultats réunis dans le tableau IV, il apparaît que la vitesse de désagrégation du comprimé augmente avec la proportion d'agent désintégrant d'enrobage sauf pour l'échantillon à 15% en poids d'agent désintégrant qui est plus faible du point de vue de la dureté.

Pour obtenir la vitesse de désagrégation recherchée, on peut utiliser une proportion d'agent désintégrant de l'ordre de 10%.

### EXEMPLE 5

Cet exemple a pour objet un comprimé multiparticulaire à libération modifiée contenant du diclofénac de sodium en tant que substance active.

En vue de l'étape d'extrusion, on prépare le mélange pulvérulent suivant:
- DFC 40 % en poids
- EC N7NF 52 % en poids
- DEP 8 % en poids.

Ce mélange pulvérulent est extrudé sur une extrudeuse monovis semi-industrielle.

Les paramètres d'extrusion sont:
- température de la zone de malaxage et de la filière: 130 à 170°C
- vitesse de la vis: 50 tours/minute
- pression régnant dans la zone de malaxage: 150 à 190 bars
- vitesse de rotation de l'outil de coupe: 7000 à 8000 tours/minute
- diamètre de l'orifice de la filière: 1 mm.

Les sphéroïdes obtenus sont utilisés pour la préparation de comprimés multiparticulaires dosés à 100 mg de DFC.

On procède d'abord à l'enrobage des sphéroïdes.

Pour ce faire, les sphéroïdes sont disposés dans une turbine.

On leur applique tout d'abord un liant constitué par une solution alcoolisée de la polyvinylpyrrolidone commercialisée sous la désignation PVP K30 par la Société BASF.

Sur les sphéroïdes ainsi traités, on applique un agent désintégrant, à savoir celui commercialisé par la Société BASF sous la marque KOLLIDON CLM, en une proportion de 10% en poids d'agent désintégrant par rapport à la masse du sphéroïde.

A partir des sphéroïdes ainsi constitués, on prépare des comprimés multiparticulaires par compression sur une machine à comprimer rotative vendue sous la marque SVIAC PR 6 par la Société SVIAC en utilisant des poinçons plats de 10 mm de diamètre.

Pour les comprimés ainsi obtenus, on détermine les paramètres rapportés dans le tableau V.

**TABLEAU V**

| Paramètres | Comprimé multiparticulaire dosé à 100 mg de DFC selon l'invention |
|---|---|
| Titre (mg/g) | 357,9 |
| Masse moyenne (mg) | 279,4 ± 3,6 |
| Dureté (N) | 45,10 ± 5,63 |
| Epaisseur (mm) | 3,58 ± 0,03 |
| Friabilité (%) | 0,09 |
| Temps de désagrégation | 9 min 44 s |

Ces comprimés sont soumis à une étude pharmacocinétique chez l'homme sain (six sujets).

Les résultats des principaux paramètres pharmacocinétiques observés chez l'homme sain après administration d'un comprimé multiparticulaire de DFC dosé à 100 mg de substance active, à savoir:
- le tmax qui désigne le temps, exprimé en heures, au bout duquel la concentration sérique en principe actif atteint un maximum,
- le Cmax (µg/l) qui désigne la valeur de la concentration maximale en principe actif atteinte à la valeur tmax; cette concentration s'exprime en µg de principe actif par litre de sérum,
- la AUC 0-12 heures (µg.h/l) qui désigne la surface sous courbe des concentrations sériques du principe actif en fonction du temps jusqu'au dernier prélèvement quantifié, c'est-à-dire 12 heures,
sont réunis dans le tableau VI ci-après:

**TABLEAU VI**

| Paramètres | Comprimé multiparticulaire dosé à 100 mg de DFC selon l'invention |
|---|---|
| tmax (h) | 1,25 |
| Cmax (µg/l) | 372 ± 58 |
| AUC 0-12 heures (µg.h/l) | 1176 ± 163 |

L'examen des résultats réunis dans le tableau VI permet de vérifier que la désagrégation du comprimé suivie de la dissolution et de l'absorption du Diclofénac de Na contenu dans les sphéroïdes s'effectue bien dans la partie haute du tube digestif du sujet adulte sain. La faible variabilité entre les sujets démontre la bonne qualité de la formulation de DFC selon l'invention.

## Revendications

1. Procédé de fabrication de particules, notamment de sphéroïdes de diamètre constant compris entre 0,5 et 2 mm, comportant une substance active et présentant une courbe de libération de la substance active contrôlée et prédéterminée et qui comporte successivement :
- une étape de sélection d'une substance active et d'un matériau thermoplastique, comportant au moins un excipient de nature polymère et au moins un agent plastifiant,
- une étape de mélange de la substance active et du matériau thermoplastique,
- une étape d'extrusion sans-solvant du mélange à température contrôlée fournissant au moins un fil ou profilé extrudé,
- une étape de découpage du profité en particules élémentaires,
**caractérisé par le fait qu'**il comporte une étape de mûrissement du mélange de la substance active et du matériau thermoplastique avant extrusion à une température et pendant une durée choisies en fonction des caractéristiques de la courbe de libération du principe actif visé et comprises respectivement entre 20 et 70° C et entre 30 minutes et 150 heures, ce grâce à quoi il devient possible, d'une part, dé stocker le mélange de substance active et de matériau thermoplastique jusqu'à 7 jours avant l'extrusion et, d'autre part, d'apporter pour une courbe de libération donnée une quantité accrue de substance active sans augmentation de volume du comprimé.

2. Installation pour la fabrication de sphéroïdes qui sont le résultat du découpage du fil ou profilé obtenu par extrusion à chaud sans solvant du mélange d'une substance active et d'un matériau thermoplastique et de l'enrobage des particules résultant du découpage par un revêtement comportant au moins un agent désintégrant et pouvant également comporter un agent liant et éventuellement une substance active, ladite installation étant **caractérisée par le fait qu'**elle est constituée par une extrudeuse comportant, à la sortie de la filière d'extrusion, un outil de coupe du fil ou profilé extrudé équipé de couteaux présentant la forme qui résulté des figures 1 et 2.

## Patentansprüche

1. Verfahren zum Herstellen von Partikeln, insbesondere von Sphäroiden von konstantem, zwischen 0,5 und 2 mm liegendem Durchmesser, die eine aktive Substanz enthalten und eine gesteuerte und vorbestimmte Kurve der Freisetzung der aktiven Substanz aufweisen, welches Verfahren nacheinander umfasst:
- einen Schritt der Auswahl einer aktiven Substanz und eines thermoplastischen Materials, das mindestens ein Bindemittel polymerer Natur und mindestens einen Weichmacher-Wirkstoff enthält,
- einen Schritt des Mischens der aktiven Substanz und des thermoplastischen Materials,
- einen Schritt des lösemittelfreien Extrudierens der Mischung bei gesteuerter Temperatur, der mindestens einen extrudierten Strang oder ein extrudiertes Profil liefert,
- einen Schritt des Abschneidens des Profils in elementare Partikel,
**gekennzeichnet durch** die Tatsache, dass es einen Schritt des Reifens der Mischung aus der aktiven Substanz und des thermoplastischen Materials vor dem Extrudieren bei einer Temperatur und während einer Zeitdauer umfasst, die abhängig von der Kurve der Freisetzung des jeweiligen aktiven Wirkstoffs ausgewählt werden und die jeweils zwischen etwa 20 und 70°C und zwischen 30 Minuten und 150 Stunden liegen, dank dessen es möglich wird, einerseits die Mischung aus aktiver Substanz und thermoplastischem Material bis zu 7 Tage vor der Extrusion zu lagern und andererseits für eine gegebene Kurve der Freisetzung eine erhöhte Menge an aktiver Substanz ohne Vergrößerung des Volumens der Tablette zu schaffen.

2. Anlage zum Herstellen von sphäroidförmigen Körpern, die das Ergebnis des Abschneidens des Strangs oder des Profils sind, die durch Extrusion bei Wärme ohne Lösemittel der Mischung einer aktiven Substanz und eines thermoplastischen Materials erhalten werden, und der Umhüllung der sich aus dem Abschneiden ergebenden Partikel durch eine Beschichtung, die mindestens einen Zersetzungswirkstoff umfasst und auch ein Bindemittel und ggf. eine aktive Substanz enthalten kann, wobei besagte Anlage **dadurch gekennzeichnet ist, dass** sie von einem Extruder gebildet ist, der am Ausgang der Extrusionsdüse ein Werkzeug zum Schneiden des Strangs oder Profils umfasst, welches mit Messern ausgestattet ist, die die sich aus den Fig. 1 und 2 ergebende Form aufweisen.

## Claims

1. A process for producing particles, especially spheroids with a constant diameter in the range 0.5 to 2 mm, comprising an active substance and having a controlled and predetermined active substance release curve and which comprises, in succession:
• a step for selecting an active substance and a thermoplastic material comprising at least one excipient of a polymeric nature and at least one plasticizing agent;
• a step for mixing the active substance and the thermoplastic material;
• a step for solvent-free extrusion of the mixture at a controlled temperature to produce at least one extruded filament or profile;
• a step for cutting the profile into elementary particles;
**characterized in that** it comprises a step for maturing the mixture of the active substance and the thermoplastic material prior to extrusion at a temperature and for a period that are selected as a function of the characteristics of the target active substance release curve and in the range 20°C to 70°C and 30 minutes to 150 hours respectively, by means of which it becomes possible to store the mixture of the active substance and the thermoplastic material for up to 7 days prior to extrusion, and to provide, for a given release curve, an increased quantity of active substance without increasing the volume of a tablet.

2. A unit for producing spheroids which are the result of cutting a filament or profile obtained by hot solvent-free extrusion of a mixture of an active substance and a thermoplastic material and coating the particles resulting from cutting with a coating comprising at least one disintegrating agent and which may also comprise a binder and an optional active substance, said unit being **characterized in that** it is constituted by an extruder comprising, at the outlet from the extrusion die, a tool for cutting an extruded filament or profile provided with knives having the shape shown in Figures 1 and 2.
